# EUROPEAN PATENT APPLICATION

(11) **EP 4 834 268 A1**
(43) Date of publication of application: **04.11.2026**
(21) Application number: 26175930.2
(22) Date of filing: 30.04.2026
(51) Int. Cl.: A61B 34/20, A61B 34/30, A61B 46/10, A61B 90/00

(54) **ROBOTIC SURGERY SYSTEM WITH FIELD GENERATOR**

(30) Priority: 30.04.2025 US 202563834885 P
(71) Applicant: Automated Intelligent Robotics LLC, Sarasota, Florida 34233 (US)
(72) Inventor: Sweeney II, Thomas Medrick, Myakka City, 34251 (US); Sweeney III, Thomas Medrick, San Diego, 92130 (US); Kuczynski, John Douglas, Sarasota, 34235 (US)
(74) Representative: Cyrson, Matthew Dominic

(57) **Abstract**

An exemplary robotic surgical system (10) includes a robot arm (12), a field generator (14), a field sensor, and a controller (18). The field generator (14) is coupled to the robot arm (12) for generating a magnetic or electromagnetic field. The field sensor is coupled to a trackable object trackable within the magnetic or electromagnetic field. The controller (18) determines a position of the trackable object in relation to the robot arm (12) based on data from the field sensor. The robotic surgical system (10) may also include a camera configured for optical tracking the trackable object, wherein the controller (18) is operatively in communication with the camera and the field sensor, and configured to determine the position of the trackable object in relation to the robot arm (12) based on data received from at least one of the field sensor and the camera. The robot arm (12) may also attach to an end effector (24) separate from or integral with the field generator (14).

## Description

### FIELD OF THE INVENTION

The subject disclosure relates generally to robotic surgical systems, and more particularly to surgical robotic surgical systems with end effectors and tracking systems.

### BACKGROUND

Surgical navigation, the technique of guiding surgical instruments with precision using imaging technologies, has a history rooted in both medical innovation and technological advancement. Its origins can be traced back to the late 20th century, when advances in computer science and medical imaging converged. The development of CT and MRI in the 1970s and 1980s enabled physicians to visualize internal structures in unprecedented detail. These imaging tools laid the foundation for early image-guided surgery systems in the 1990s.

Initially used in neurosurgery- where precision and accuracy are critical and the anatomy is relatively stable - surgical navigation systems helped surgeons localize brain tumors and plan surgical paths more accurately. These systems evolved with the integration of real-time tracking, robotics, and augmented reality, leading to broader adoption in orthopedic, spinal, ENT, and even minimally invasive surgeries.

Today, surgical navigation continues to evolve with advances in artificial intelligence, intraoperative imaging, and robotic-assisted systems, improving outcomes by increasing precision and accuracy, reducing invasiveness, and enhancing safety across a range of surgical disciplines. Today's systems incorporate artificial intelligence, improved haptic feedback, and greater autonomy. Surgical robotics continues to evolve, blending human judgment with robotic precision, shaping a future where surgery is safer, faster, and more precise than ever before. While advancements have been made, problems persist with current navigation and robotic surgical systems. For example, system accuracy and precision of the placement of prosthesis, e.g., spinal pedicle screw placement, continues to fail to meet user needs.

### SUMMARY

The following presents a simplified summary in order to provide a basic understanding of some aspects of one or more exemplary embodiments or examples of the present teachings. This summary is not an extensive overview, nor is it intended to identify key or critical elements of the present teachings, nor to delineate the scope of the disclosure. Rather, its primary purpose is merely to present one or more concepts in simplified form as a prelude to the detailed description presented later. Additional goals and advantages will become more evident in the description of the figures, the detailed description of the disclosure, and the claims.

In accordance with an exemplary embodiment of the subject disclosure, there is provided a robotic surgical system including a robot arm, a field generator for generating a magnetic or electromagnetic field, a field sensor coupled to a trackable object trackable within the magnetic or electromagnetic field, and a controller configured to determine a position of the trackable object in relation to the robot arm based on data from the field sensor.

According to an aspect, the robotic surgical system further includes a camera configured for optical tracking of the trackable object and operatively in communication with the controller. According to another aspect, the controller is configured to determine the position of the trackable object in relation to the robot arm based on data received from at least one of the field sensor and the camera. According to another aspect, the field sensor includes an optical fiducial. According to another aspect, the field generator includes a central aperture. According to another aspect, the robot arm includes an end effector.

According to aspect, the robotic surgical system also includes a second sensor coupled to a second trackable object, such that one of the field sensor and the second sensor is operatively attached to a tool, and the other one of the field sensor and the second sensor is configured for attachment to a patient's bone. According to another aspect, the controller is configured to track a position of the tool relative to the patient's bone.

According to an aspect, the robotic surgical system includes an end effector. According to another aspect, the end effector includes a mounting nozzle coupled with a sterile drape retained by a drape cap. According to another aspect, the end effector includes a housing, a gear train within the housing, and a tool holder operatively connected to the gear train for rotational movement relative to the housing. According to another aspect, the end effector includes a tool holder holding a tool coupled at a distal end of the end effector.

In accordance with an aspect, the robotic surgical system includes a first robot arm having a field generator for generating a magnetic or electromagnetic field, and a second robot arm. According to another aspect, the controller is operatively in communication with the first robot arm, the second robot arm and the field sensor to determine a position of the trackable object in relation to the first and second robot arms based on data received from the sensor.

According to an aspect, the robotic surgical system also includes a camera configured for optical tracking of the trackable object. According to another aspect, the controller is configured to determine a position of the trackable object in relation to the robot arm based on data received from at least one of the sensor and the camera. According to another aspect, the controller is configured to determine a position of the trackable object in relation to the first and second robot arms based on data received from at least one of the sensor and the camera.

According to an aspect, the second robot arm includes an end effector, and the field generator includes a central aperture. According to another aspect, the trackable object is a surgical retractor. According to another aspect, the trackable object is at least one of a surgical retractor, a spinous process tracking pin, an iliac tracking pin, an end effector, a tool, a spinous process, an ilium, and a vertebral body. According to another aspect, the second robot arm includes a tool. According to another aspect, a second sensor is attached to the tool. According to another aspect, the sensor coupled to the trackable object is configured for attachment to a patient's bone, and the controller is configured to track the position of the tool relative to the patient's bone.

According to other aspects illustrated herein, the surgical robotics system includes a second 7-axis robot where the second robot has a permanent-magnet based field generator mounted to the robot. According to other aspects, the second robot has an electromagnetic field generator mounted to the flange of the robot. The second robot positions the field generator in an ideal location for tracking relative to a patient, for example, a patient's spine being worked on by the first robot.

According to other aspects, the robotics system may include infrared camera-based or optical navigation combined with the robot and EM navigation system to track position of the overall surgical working area while the EM navigation system is used to track the local position of the working tool being used and/or adjacent vertebral bodies.

The foregoing and/or other aspects and utilities embodied in the present disclosure may also be achieved by providing a robotic surgical system including a surgical robotics system combined with an end effector and a permanent magnetic surgical navigation system, where the field generator component of the navigation system is coupled to or incorporated into the robot. The field generator is positioned so that the functional volume of the magnetic field generated encompasses the tool to be guided and clinical working area. Thus, the surgical robotics system can be used as an integral part or an attachment to the end effector to register and track the end effector and instruments in the spine and pelvis using bony fiducial attachments.

According to yet other aspects, the robotic surgical system includes a surgical robotics system combined with an electromagnetic surgical navigation system, where the field generator component of the EM navigation system is coupled to or incorporated into the robot. The field generator can be in line with the access of the working or surgical tools, allowing work through a portal. For example, the EM field generator may be placed so that the tool to be guided passes through an opening in the field generator. The surgical robotics system may alternatively be combined with a permanent magnetic surgical navigation system, where the field generator component of the navigation system is coupled to or integrated into the robot. An infrared camera-based navigation system may be combined with the robot and permanent magnet/electromagnetic surgical navigation system to track position of the overall surgical working area while the permanent magnet navigation system is used to track the local position of the working tool being used and/or adjacent vertebral bodies.

According to still other aspects, the robotic surgical system includes a surgical robotics system and an end effector combined with a second robot and an electromagnetic surgical navigation system, where the field generator component of the EM navigation system is coupled to or integrated into the second robot. The EM field generator is positioned so that the tool attached to the first robot end effector passes through an opening in the field generator. In other words, the EM field generator may be used on a separate robot arm than the end effector or magnetic field generator, and can include a surgical exposure device such as a constant diameter tube, expandable tube, or expandable multipart retractor, or an attachment integral to the end effector such that it is not in the direct working axis of the end effector which will facilitate drilling, tapping and screw placement by registration and tracking.

In embodiments of the subject disclosure, the robotic surgical system and magnetic/electromagnetic field-based navigation system may be paired with an optical navigation system, and a processing device or controller operatively in communication with the robotic surgical system and navigation systems thereof to operate the robotic surgical system with greatest accuracy and precision. The navigation systems may track sensors or fiducials attached to hand-held tools, robotically controlled tools, the patient's skin, the patient's bone including vertebral bodies, pelvis, or other stable structure. Multiple objects may be tracked simultaneously, and the position of the field generator, robot arms, and tools may be optimized for the object being tracked or worked on. It is understood that the electromagnetic field generator and sensors are interchangeable with permanent-magnet field generator and sensors.

Other features and advantages of the subject disclosure will be apparent from the following more detailed description of the exemplary embodiments.

### BRIEF DESCRIPTION OF THE DRAWINGS

The foregoing summary, as well as the following detailed description of the exemplary embodiments of the surgical robotic surgical system, will be better understood when read in conjunction with the appended drawings. For the purpose of illustrating the surgical robot system, there are shown in the drawings exemplary embodiments of the subject disclosure. It should be understood, however, that the robotic surgical system is not limited to the precise arrangements and instrumentalities shown.
FIG. 1 is a perspective view of a robotic surgical system in accordance with an exemplary embodiment of the subject disclosure;
FIG. 2 is a partial perspective view of the robotic surgical system of FIG. 1;
FIG. 3 is a partial perspective view partially exploded of the robotic surgical system of FIG. 1;
FIG. 4 is an exploded perspective exploded view of an end effector of the robotic surgical system of FIG. 1;
FIG. 5 is a perspective view of a drape cutter of the robotic surgical system of FIG. 1;
FIG. 6 is a longitudinal cross-sectional view of the drape cutter of FIG. 5;
FIG. 7 is another perspective view of the drape cutter of FIG. 5;
FIG. 8 is an enlarged perspective view of a field generator of the robotic surgical system of FIG. 1;
FIG. 9 is a perspective view of the robot arm and end effector of the robotic surgical system of FIG. 1;
FIG. 10 is another partial perspective view of the robotic surgical system of FIG. 1;
FIG. 11 is a perspective view of exemplary fiducials applicable to the robotic surgical system of FIG. 1;
FIG. 12 is a perspective view of a robotic surgical system in accordance with another exemplary embodiment of the subject disclosure;
FIG. 13 is a perspective view of a robotic surgical system in accordance with yet another exemplary embodiment of the subject disclosure;
FIG. 14 is a partial perspective view of the robotic surgical system shown in FIG. 13;
FIG. 15 is a perspective view of a robotic surgical system in accordance with still another exemplary embodiment of the subject disclosure;
FIG. 16 is a partial perspective view of the robotic surgical system of FIG. 15;
FIG. 17 is another partial perspective view of the robotic surgical system of FIG. 15;
FIG. 18 is a flowchart depicting the operation of robotic surgical system in accordance with another exemplary embodiment of the subject disclosure;
FIG. 19 is a flowchart depicting the operation of robotic surgical system in accordance with another exemplary embodiment of the subject disclosure;
FIG. 20 is a flowchart depicting the operation of robotic surgical system in accordance with another exemplary embodiment of the subject disclosure; and
FIG. 21 is a flowchart depicting the operation of robotic surgical system in accordance with another exemplary embodiment of the subject disclosure.

### DETAILED DESCRIPTION

Reference will now be made in detail to the various exemplary embodiments of the subject disclosure illustrated in the accompanying drawings. Wherever possible, the same or like reference numbers will be used throughout the drawings to refer to the same or like features. It should be noted that the drawings are in simplified form and are not drawn to precise scale.

Certain terminology is used in the following description for convenience only and is not limiting. Directional terms such as top, bottom, left, right, above, below and diagonal, are used with respect to the accompanying drawings. The term "distal" shall mean away from the center of a body. The term "proximal" shall mean closer towards the center of a body and/or away from the "distal" end. The words "inwardly" and "outwardly" refer to directions toward and away from, respectively, the geometric center of the identified element and designated parts thereof. Such directional terms used in conjunction with the following description of the drawings should not be construed to limit the scope of the subject disclosure in any manner not explicitly set forth. Additionally, the term "a," as used in the specification, means "at least one." The terminology includes the words above specifically mentioned, derivatives thereof, and words of similar import.

"About" as used herein when referring to a measurable value such as an amount, a temporal duration, and the like, is meant to encompass variations of ±20%, ±10%, ±5%, ±1%, or ±0.1% from the specified value, as such variations are appropriate.

"Substantially" as used herein shall mean considerable in extent, largely but not wholly that which is specified, or an appropriate variation therefrom as is acceptable within the field of art. "Exemplary" as used herein shall mean serving as an example.

Throughout this disclosure, various aspects of the subject disclosure can be presented in a range format. It should be understood that the description in range format is merely for convenience and brevity and should not be construed as an inflexible limitation on the scope of the subject disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

The term "controller" or "control system" is used herein generally to describe various apparatus such as a computing device relating to the operation of one or more device that directs or regulates a process or machine. A controller can be implemented in numerous ways (*e.g.,* such as with dedicated hardware) to perform various functions discussed herein. A "processor" is one example of a controller which employs one or more microprocessors that may be programmed using software (*e.g.,* microcode) to perform various functions discussed herein. A controller may be implemented with or without employing a processor, and also may be implemented as a combination of dedicated hardware to perform some functions and a processor (*e.g.,* one or more programmed microprocessors and associated circuitry) to perform other functions. Examples of controller components that may be employed in various embodiments of the present disclosure include, but are not limited to, conventional microprocessors, application specific integrated circuits (ASICs), field-programmable gate arrays (FPGAs) and the like.

Embodiments as disclosed herein may also include computer-readable media for carrying or having computer-executable instructions or data structures stored thereon. Such computer readable media can be any available media that can be accessed by a general purpose or special purpose computer. By way of example, and not limitation, such computer-readable media can include RAM, ROM, EEPROM, CD-ROM or other optical disk storage, magnetic disk storage or other magnetic storage devices, or any other medium which can be used to carry or store desired program code means in the form of computer-executable instructions or data structures.

When information is transferred or provided over a network or another communications connection (either hardwired, wireless, or combination thereof) to a computer, the computer properly views the connection as a computer-readable medium. Thus, any such connection is properly termed a computer-readable medium. Combinations of the above should also be included within the scope of the computer-readable media.

Computer-executable instructions include, for example, instructions and data which cause a general-purpose computer, special purpose computer, or special purpose processing device to perform a certain function or group of functions. Computer-executable instructions also include program modules that are executed by computers in stand-alone or network environments. Generally, program modules include routines, programs, objects, components, and data structures, and the like that perform particular tasks or implement particular abstract data types. Computer-executable instructions, associated data structures, and program modules represent examples of the program code means for executing steps of the methods disclosed herein. The particular sequence of such executable instructions or associated data structures represents examples of corresponding acts for implementing the functions described therein.

Although embodiments of the subject disclosure are not limited in this regard, discussions utilizing terms such as, for example, "processing," "tracking," "computing," "calculating," "determining," "using," "establishing", "analyzing", "checking", or the like, may refer to operation(s) and/or process(es) of a controller, computer, computing platform, computing system, or other electronic computing device, that manipulate and/or transform data represented as physical (*e.g.,* electronic) quantities within the computer's registers and/or memories into other data similarly represented as physical quantities within the computer's registers and/or memories or other information storage medium that may store instructions to perform operations and/or processes.

Furthermore, the described features, advantages and characteristics of the exemplary embodiments of the subject disclosure may be combined in any suitable manner in one or more embodiments. One skilled in the relevant art will recognize, in light of the description herein, that the present disclosure can be practiced without one or more of the specific features or advantages of a particular exemplary embodiment. In other instances, additional features and advantages may be recognized in certain embodiments that may not be present in all exemplary embodiments of the subject disclosure.

The term "end effector" is used herein generally to describe a tool or instrument configured for attachment to a robotic manipulator's distal mechanical interface and configured to interact with tissue, instruments, or the surgical environment (*e.g.,* by grasping, cutting, suturing, sealing, stapling, imaging, sensing, suction/irrigation, energy delivery, etc.). While not being limited to a particular theory, an end effector is a device mounted to the distal end of the robot to perform a function. For example, an end effector is a tool or device attached at the robot's distal mechanical interface (*e.g.,* the "wrist") that interacts with the environment and manipulates patient tissue or anatomy. In embodiments of the subject disclosure, exemplary end effectors may include a gripper, a retractor, needle drivers, scissors, staplers, surgical instrument, a cutting tool and the like. As such, an end effector may include the surgical instrument driven by the robot that performs the task at the operative field, which includes the entirety of the area of interest, *e.g.,* the entire spine, or the localization of a single vertebral level or soft tissue localization. The end effector may also be referred to as End of Arm Tooling (EOAT).

An end effector can include or carry trackable elements (*e.g.,* optical fiducials, electromagnetic sensors, surgical tools) for real-time position estimation of trackable element or instrument attached to the trackable element relative to patient anatomy and the robot base. In some embodiments, an end effector may refer specifically to a terminal functional portion of an instrument (*e.g.,* jaws, electrodes, stapling cartridge) exclusive of coupling. In some embodiments, an end effector may encompass a wrist or angulation mechanism, drive transmissions, and/or sensors disposed at or adjacent a tool distal tip. An end effector may also include an attached or attachable field generator that can be used to register and track trackable elements within the operative field. According to aspects of the exemplary embodiments, registration may include placing EM sensors on the patient anatomy (spinous processes, pelvis or any other bony spinal anatomy) to be tracked. EM sensor locations will be captured by intraoperative CT scan or MRI, processing the image to locate EM sensors relative to the operative field. As an alternative, a preoperative CT scan or MRI can be registered to intraoperative fluoroscopic x-ray images after the EM fiducials have been placed.

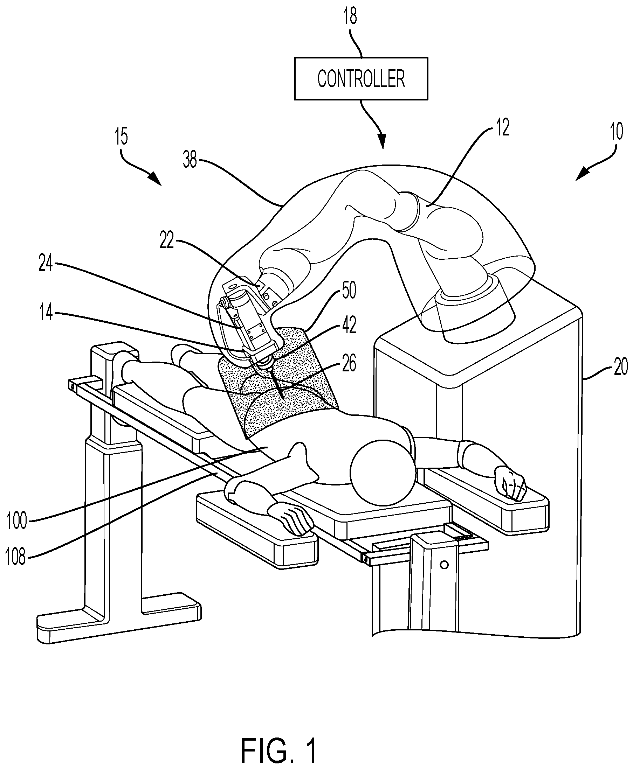

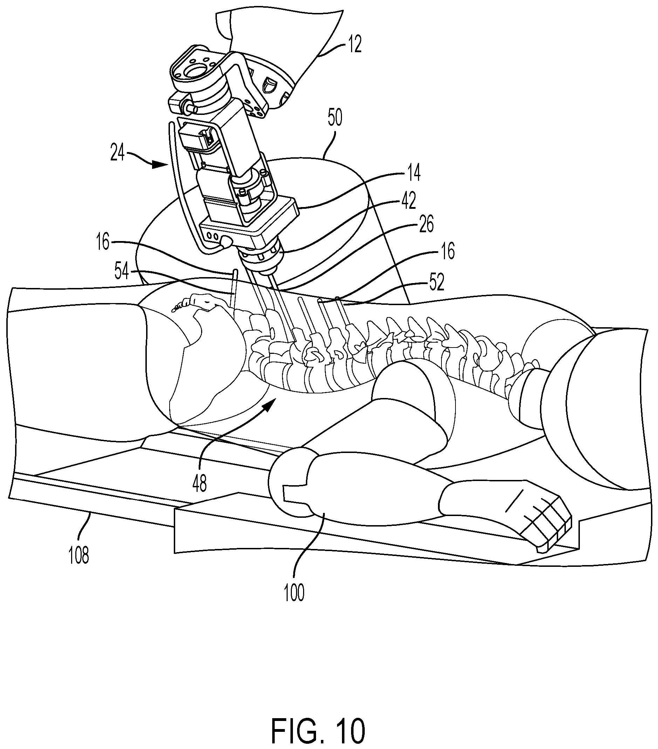

FIGS. 1-4 illustrate a robotic surgical system 10 in accordance with an exemplary embodiment of the subject disclosure. The robotic surgical system includes a robot arm 12, a field generator 14, a field sensor 16 (FIG. 10) and a controller 18. The robot arm may be designed for precise positioning and manipulation of surgical instruments about a patient. The robot arm 12 includes a proximal end coupled to a robot base 20 which may be fixed or selectively movable, such as by wheels, within an operating room. The robot arm 12 also includes a distal end 22 disposed opposite the proximal end about which an end effector 24 can be coupled to.

Between the proximal end and distal end, the robot arm 12 includes a series of interconnected structural linkages, such as segments and joints arranged to provide multiple degrees of freedom, allowing the arm to bend, rotate, and pivot over a wide range of controlled motions, while maintaining stability and accuracy during medical procedures. The robot arm 12 further includes one or more actuators, drives and/or motors which are configured to be controlled by a computer having the controller 18 to move the robot arm to desired positions. As such, the robot arm is an intermediary interface having adjustable and fixed supports between the robot base 20 and its distal end 22.

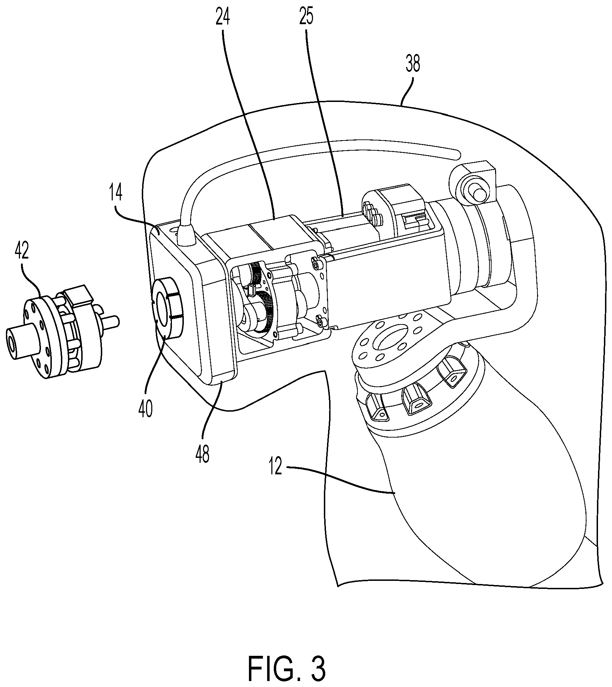

The field generator 14 is configured as best shown in FIGS. 3, 4 and 8 and may include a non-magnetic, sterilizable housing 48 and at least one of magnetic circuit elements (not shown) and a coil assembly (not shown) of at least one wound conductor in the housing. The field generator is coupled to and operatively associated with the robot arm. More particularly, the field generator 14 can be coupled to the distal end 22 or about the distal end of the robot arm 12. Owing to this configuration, a field can be generated about the distal end of the robot arm.

While not being limited to a particular theory, the field generator 14 can be a magnetic field generator or an electromagnetic (EM) field generator for generating a magnetic field or an electromagnetic field, respectively. In some exemplary embodiments, the field generator can include a permanent magnet as a magnetic circuit element providing a magnetic field. In select examples, the field generator includes a transmitter having a coil assembly (*e.g.,* solenoidal coils, axial/uniform field coils, gradient producing coils, saddle coils, etc.) of at least one wound conductor in the housing that emits a low-frequency electromagnetic field in tracking space adjacent the patient. The generated magnetic or electromagnetic field can span an entire operative field or surgical working area.

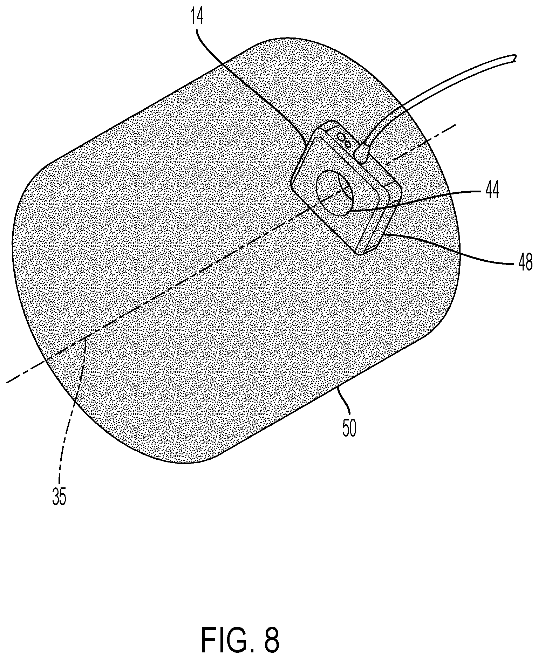

In accordance with certain aspects of the subject disclosure, the field generator 14 further includes a central aperture 44 (FIG. 8). The central aperture may be mounted on the end-effector mounting nozzle 36. While not being limited to a particular theory, the central aperture 44 is disposed so as to be generally and/or substantially coaxial about the central longitudinal axis of the end effector 24 or generally and/or substantially coaxial about a central longitudinal axis of the mounting nozzle 28 of the end effector. The central aperture 44 is also configured to be generally and/or substantially coaxial about a centerline 35 of the field generated by the field generator. Thus, the central aperture allows for the field generated by the field generator to be in-line with tools attached to the end effector 24 or with the access or attachment mechanism for the tools to the end effector.

The central aperture 44 may beneficially provide a region of field uniformity, as coil assembly geometries produce their most homogeneous field in the central aperture. In addition, the central aperture keeps conductive/magnetic structure away from the center, and lowers eddy currents and magnetic distortion near a region of interest, thereby improving electromagnetic sensing and tracking. Moreover, the field generator 14 may be positioned with the central aperture 44 adjacent about the mounting nozzle 36 such that a sterile drape 38 can seal around the field generator 14, isolating the non-sterile field generator, end effector 24 and robot arm 12 from the sterile clinical area.

In alternative exemplary embodiments, the field generator 14 can be attached to a distal end or about the distal end of an end effector. Additionally, in accordance with an alternative aspect of the subject disclosure, the field generator may be integrally formed as a part of the end effector 24 or the robot arm 12.

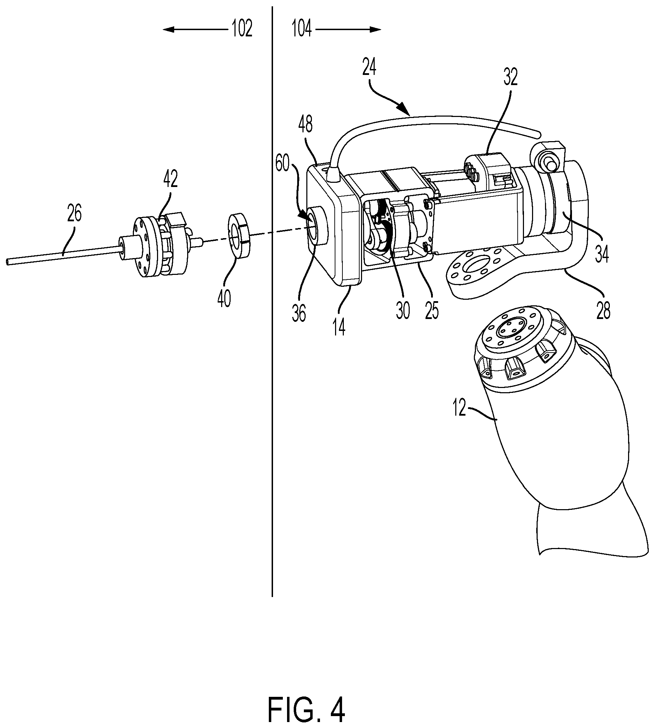

An exemplary end effector 24 in accordance with the subject disclosure is configured as best shown in FIG. 4. The end effector 24 includes a housing 25 and a mounting nozzle or bracket 28. The end effector is attached to the distal end 22 of the robot arm 12 via the mounting bracket. As such, the end effector 24 may be attached to, integrated with, or integrated into the robot arm 12. The end effector 24 also includes a gear train 30, a motor 32, such as a servo-motor, and a transducer 34 (*e.g.,* force and torque transducer) mounted within the housing. About a distal end of the end effector is a mounting nozzle 36 for attaching a tool thereto. The mounting nozzle 36 can be configured as an annular pipe or cylinder having an axially aligned through-hole 60 for external mechanical access to the gear train or motor within the end effector housing.

In another exemplary embodiment, the end effector may include a tool holder 42 coupled to the end effector via the mounting nozzle 36 (FIG. 4). The tool holder 42 may be operatively connected to the gear train 30 of the end effector for movement therewith (*e.g.,* rotational movement relative to the end effector housing). The tool holder may attach to a surgical tool 26, such that the surgical tool is operatively connected to the gear train for powering the surgical tool. While the tool holder 42 is shown coupled to the end effector 24 via the mounting nozzle 36, it is understood that the tool holder may also be coupled to the end effector through a drape cap 400(as further discussed below).

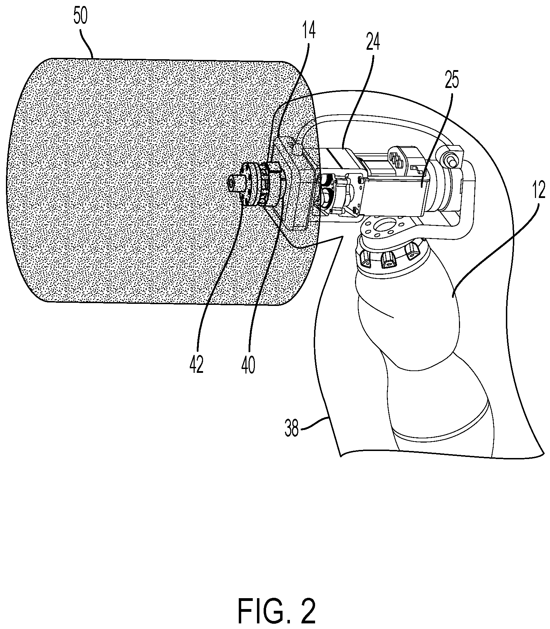

Referring to FIGS. 2-4, when in operation for a surgical procedure, the mounting nozzle 36 may be coupled to a sterile drape 38, which is retained on the mounting nozzle by a drape cap 40. The sterile drape 38 is placed over the end effector 24, the robot arm 12, and surrounding surfaces of the robot arm to establish and maintain a sterile field around the surgical site. For example, the sterile drape 38 can maintain a sterile field 102 separate from a non-sterile field 104 behind the drape that covers the end effector 24 and robot arm 12.

The drape cap 40 is an annular cap configured to seal the sterile drape 38 to the mounting nozzle 36. The drape cap 40 may include an outer annular wall with finger tabs 45 extended towards a proximal end of the cap. The finger tabs 45 may expand and contract radially to fit around and attach to the mounting nozzle. When the drape cap is attached to the mounting nozzle, the drape cap secures the sterile drape 38 about the perimeter of the mounting nozzle and seals the sterile drape therebetween, which separates the sterile field 102 from the non-sterile field 104.

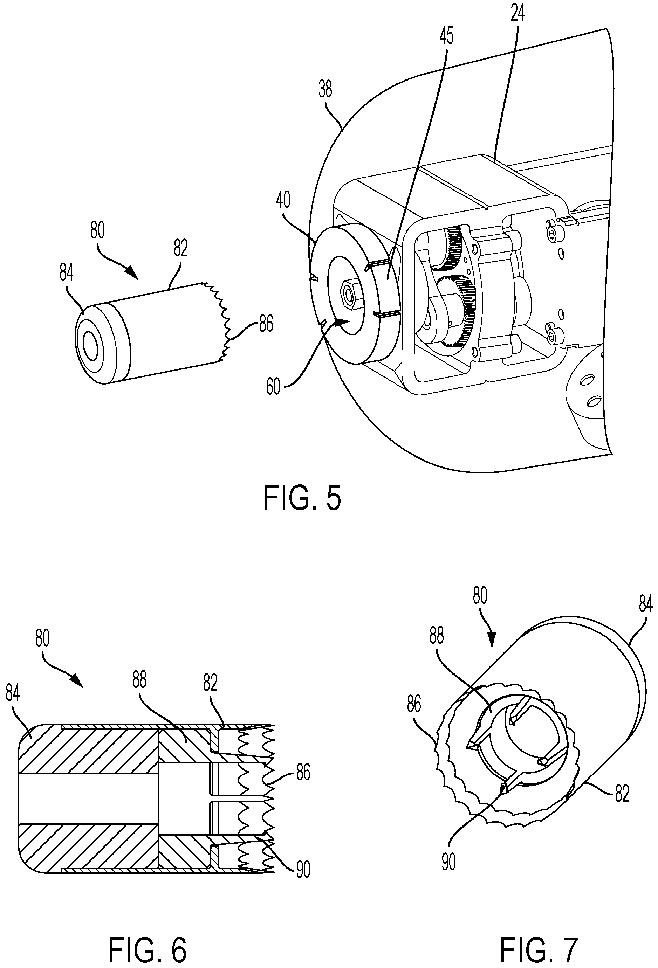

Once the sterile drape 38 is fitted over the mounting nozzle 36, a portion of the drape may cover the axial through-hole 60 within the mounting nozzle and may block access therethrough for attachment of the end effector to surgical tools. FIGS. 5-7 depict a drape cutter 80 having a cutting cylinder 82 and a push handle 84 that are coaxially aligned and rigidly attached. The cutting cylinder 82 and push handle 84 may be integrally formed. The cutting cylinder includes a proximal end with a sharp cutting edge 86 that may be serrated. The cutting cylinder has an outer diameter less than an inner wall diameter of the drape cap 40, which allows the cutting cylinder to fit axially inside the annular drape cap.

Within the cutting cylinder 82, an inner rotor bushing 88 supports internal slug-capture barbs 90 that extend axially toward the proximal end. The barbs 90 project longitudinal from the bushing and, under axial actuation may contact the sterile drape 38 spanning the mounting nozzle through-hole 60 and cooperate with the cutting cylinder to allow rotation of the cylinder cutting edge in the through-hole to shear the sterile drape. While not being limited to a particular theory, the inner rotor bushing 88 is rotatable relative to the cutting cylinder 82. The drape cutter may be positioned under axial translation within the mounting nozzle 36. This placement may urge the barbs 90 against the sterile drape spanning the through-hole and the cutting cylinder may rotate such that the cutting edge of the drape cutter 80 engages and cuts the sterile drape 38 in the through-hole 60 of the mounting nozzle 36 for access through the mounting nozzle to the end effector 24. The cut of the sterile drape 38 does not compromise the sterile drape seal, which is maintained between the mounting nozzle and the drape cap 40.

The field sensor 16 is configured to operate within the field generated by the field generator and may be one or more of a position sensor, an optical sensor, a magnetic sensor, and a motion sensor operable within the effective volume 50. An exemplary magnetic or sensor may include a receiver having miniaturized tri-axial pickup coil assembly with mutually orthogonal inductive windings wound on high-permeability ferrite cores that detects the magnetic field and communicates with the controller 18. Exemplary optical sensors or fiducials may include passive markers (*e.g.,* retroreflective spheres and surfaces) and active markers (*e.g.,* IR LEDs).

In exemplary embodiments of the subject disclosure, the sensor 16 may be coupled (*e.g.,* adhesive, molded, soldered, press fit, embedded, sprayed, deposition) to a trackable object, such as, pins (*e.g.,* tracking pins, spinous process tracking pin 52, Iliac tracking pin 54), clamps, screws, fasteners, tools, or other structure within the operating environment that may be attached to a patient (*e.g.,* spinous process, pelvis, skin or other stable structure of the patient anatomy), tool or other structure within the operating environment. The trackable object may be a prosthesis including a spinal pedicle screw that may fixedly engage a bone of the patient *e.g.,* via threaded engagement, press-fit, or other locking approach as understood by a skilled artisan.

According to some aspects of exemplary embodiments, the pins, clamps, screws, fasteners and tools may include an optical fiducial that may be located and tracked via the optical navigation system. Some trackable objects (*e.g.,* spinous process tracking pin 52, Iliac tracking pin 54, pins, clamps, screws, fasteners, etc.) may include a field sensor therein so as to be configured for tracking within a field generated by the field generator. According to some aspects, the sensor 16 may be coupled to a trackable object like the surgical tool 26. The trackable object, when referring to a surgical tool 26, may be used through the central aperture 44 of the field generator 14, as will be described in greater detail below.

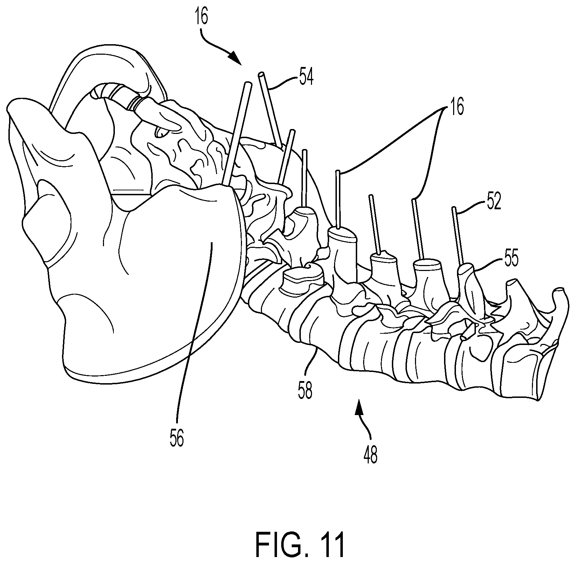

In accordance with another aspect, the robotic surgical system 10 may include a plurality of sensors 16 each configured to operate within the field (*e.g.,* effective volume 50) generated by the field generator 14 and trackable therein. The plurality of sensors 16 may be a network of sensors or may be individually accessed sensors. As can be seen in FIGS. 10 and 11, field sensors 16 may be coupled to or placed on the skin of a patient or in the bone of a patient, such as the patient's spine (*e.g.,* on a patient's ilium, spinous processes, or vertebrae), or the patient's pelvis, or other bones of the patient, as readily understood by a skilled artisan. Multiple sensors may also be used to establish spatial relationships within the operating environment (*e.g.,* between tools and patient anatomy). For example, a first field sensor may be coupled to a trackable object, such as a patient's bone, and a second field sensor may be attached to a tool trackable within the magnetic or electromagnetic field. In such a configuration, the patient's bone provides a stable anatomical reference, and the position of the tool may be tracked relative to the patient's bone via the controller 18. This enables a benefit of the robotic surgical system 10, that is, to guide or adjust the tool accurately during procedures.

The controller 18 may include, but are not limited to, one or more processors or processing units, a memory, a network interface, and a bus that communicatively couples various system components including the system memory and the network interface to the processor. The memory can include computer instructions or receive computer instructions executable by the processor to carry out the functions as described above for the robotic surgical system.

The bus represents one or more of any of several types of bus structures, including a memory bus or memory controller, a peripheral bus, an accelerated graphics port, and a processor or local bus using any of a variety of bus architectures. By way of non-limiting example, such architectures include Industry Standard Architecture (ISA) bus, Micro Channel Architecture (MCA) bus, Enhanced ISA (EISA) bus, Video Electronics Standards Association (VESA) local bus, and Peripheral Component Interconnects (PCI) bus.

The memory may include computer system readable media in the form of volatile memory, such as random-access memory (RAM) and/or cache memory. The controller 18 may further include other removable/non-removable, volatile/non-volatile computer system storage media. By way of example only, the storage system may be provided for reading from and writing to a non-removable, non-volatile magnetic media (not shown and typically called a "hard drive"). Although not shown, a magnetic disk drive for reading from and writing to a removable, non-volatile magnetic disk (*e.g.,* a "floppy disk"), and an optical disk drive for reading from or writing to a removable, non-volatile optical disk such as a CD-ROM, DVD-ROM or other optical media may be provided. In such instances, each may be connected to the bus by one or more data media interfaces. The memory may include at least one computer program product having a set (*e.g.,* at least one) of program modules that are configured to carry out the functions of embodiments of the subject disclosure.

The controller 18 may also include a program/utility, having a set (at least one) of program modules, which may be stored in the memory by way of non-limiting example, as well as an operating system, one or more application programs, other program modules, and program data. Each of the operating system, one or more application programs, other program modules, and program data or some combination thereof, may include an implementation of a networking environment. The program modules generally carry out the functions and/or methodologies of embodiments of the invention as described herein.

The controller 18 may also communicate with one or more external devices such as a keyboard, a pointing device, a display, etc.; one or more devices that enable a user to interact with the controller; and/or any devices (*e.g.,* network card, modem, etc.) that enable the controller to communicate with one or more other computing devices. Such communication may occur via the Input/Output (I/O) interfaces. Still yet, the controller may communicate with one or more networks such as a local area network (LAN), a general wide area network (WAN), and/or a public network (*e.g.,* the Internet) via the network adapter. As depicted, the network adapter communicates with the other components of the computer system via the bus. It should be understood that although not shown, other hardware and/or software components could be used in conjunction with the controller. Non-limiting examples include microcode, device drivers, redundant processing units, external disk drive arrays, Redundant Array of Independent Disk (RAID) systems, tape drives, data archival storage systems, etc.

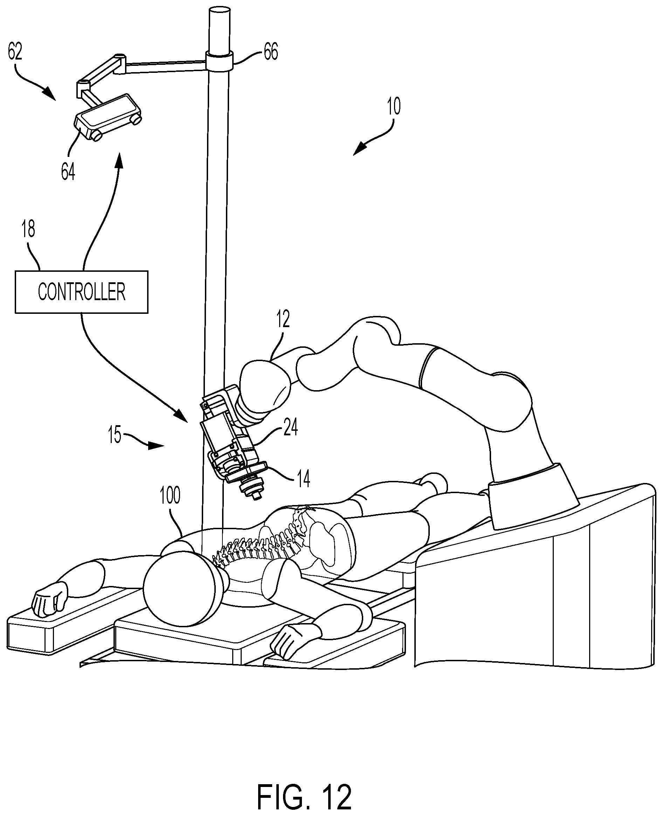

Referring to FIG. 12, in accordance with another aspect, the robotic surgical system 10 further includes a an optical tracking system 62 operatively in communication with the controller 18. The optical tracking system includes a camera 64. In other words, the robotic surgical system 10 further includes a camera 64 configured for optical tracking of *e.g.,* trackable objects and is operatively in communication with the controller. The camera 64 is configured to optically track the trackable object. The camera may be but not limited to an infrared, HD, 4K, and 3D camera. Additionally, the camera 64 may be configured to detect and/or track one or more of the sensor 16 and the trackable object, for example, when the field sensor is attached to the trackable object.

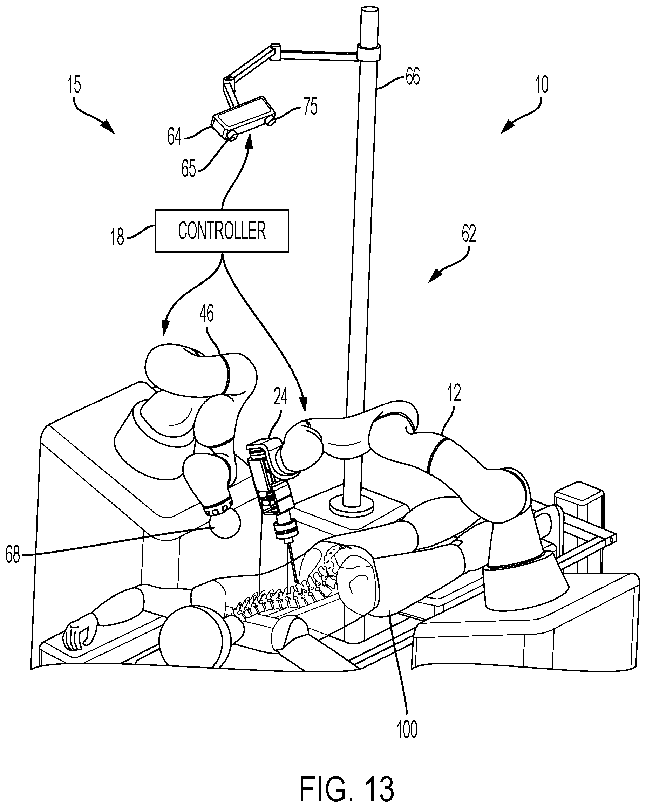

As shown in FIG. 13, the robotic surgical system 10 may include an optical device 64 having a plurality of cameras 65, 75 configured to optically track one or more of the trackable object and/or the sensor. With a second camera 75 offset from the first camera 65, the controller 18 may use triangulation to reconstruct the position and orientation of the optical sensor for real-time tracking of the trackable object. In the example shown in FIGS. 12 and 13, the optical device 64 is placed on a camera stand 66 that may be outside the field effective volume 50 (FIG. 2) generated by the field generator 14, however, it is contemplated that the cameras may operate and optically track sensors and other trackable objects within the field generated by the field generator.

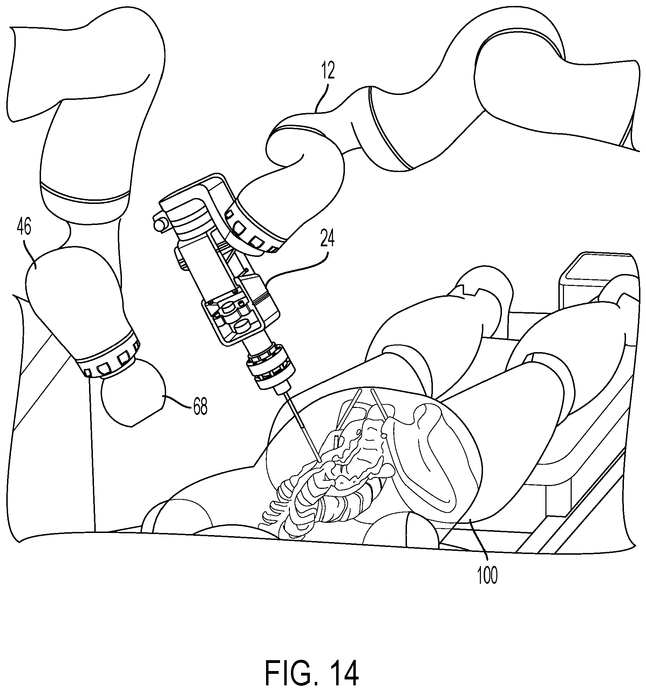

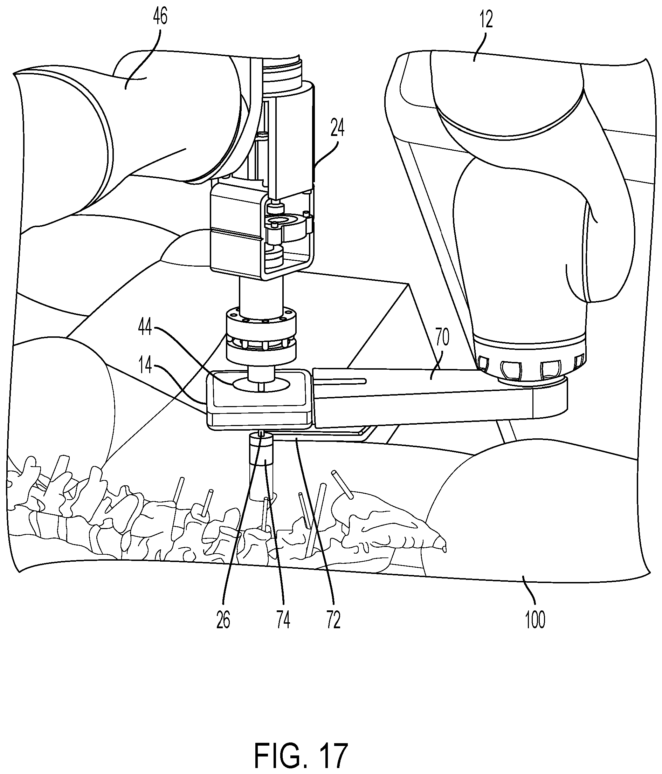

In accordance with another exemplary embodiment, the subject disclosure provides a robotic surgical system 10 that includes a plurality of robotic arms. Each of the robot arms of the robotic surgical system can include a field generator 14 adjacent its distal end. The plurality of robot arms can include the first robot arm 12 and a second robot arm 46, as shown in FIGS. 13-17. Each of the plurality of robot arms can be configured as robotic surgical systems as disclosed above (*e.g.,* having multiple axis, such as 7-axis for distal end movement with six degrees of freedom). In some aspects, one robot arm includes an end effector 24 having a tool while another robot arm has a field generator 14 for generating a magnetic field or electromagnetic field about which and wherein the robotic surgical system 10 operates.

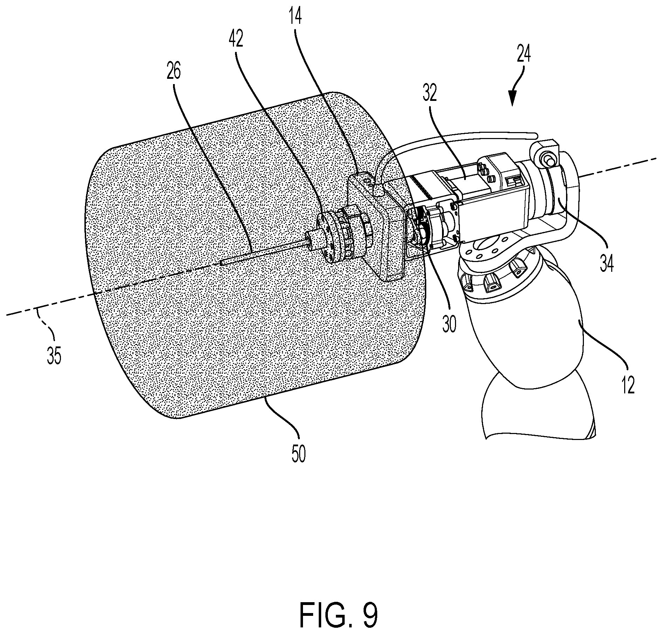

Referring to FIGS. 1, 3, 4, 8, and 9, in accordance with another exemplary aspect of the embodiment, the robotic surgical system 10 can further include a tracking system 92, for example, a magnetic or electromagnetic tracking system 15 having the field generator 14. the tracking system 92 robotic surgical system can be configured as a hybrid tracking system that includes a magnetic or electromagnetic tracking system 15 and an optical tracking system 62 to enable a global and local registration and tracking of objects and tools within the surgical field of interest. According to some aspects, the EM tracking system 15 of the end effector can be used in a hybrid capacity with an optical tracking system 62 to facilitate global (optical) and local (EM) registration and tracking. The optical or EM tracking system can also include the camera 64 on the end effector 24 for "eye in hand" visualization, and monitoring of the operative site, including assessment of structured light used to identify anatomy.

It is understood that the robotic tracking system is not limited to optical tracking systems 62 or electromagnetic tracking systems 15, as other tracking systems may also be used within the scope of the examples, such as tracking systems configured to track object via magnetic tracking, inertial tracking, ultrasonic tracking, radio frequency (RF) tracking, or Global Positioning System (GPS) tracking. A hybrid approach may allow one tracking system to maintain tracking continuity when another tracking system may be temporarily compromised by patient position, anatomical obstructions (*e.g.,* line-of-sight) or surgical procedures.

FIGS. 13-14 depict the robotic surgical system 10 having a plurality of robot arms 12, 46 with the optical tracking system 62. The plurality of robot arms includes a first robot arm 12 attached to end effector 24 having a tool 26, and the second robot arm 46 includes a field generator for generating a magnetic field or electromagnetic filed field about and within which the robotic surgical system tool 9 operates in conjunction with the first robot arm. The field generator 14 integrated with the second robot arm is a magnetic field generator 68 having a housing and a permanent magnet therein. It is understood that the field generator integrated with the second robot arm 46 may alternatively be an electromagnetic field generator.

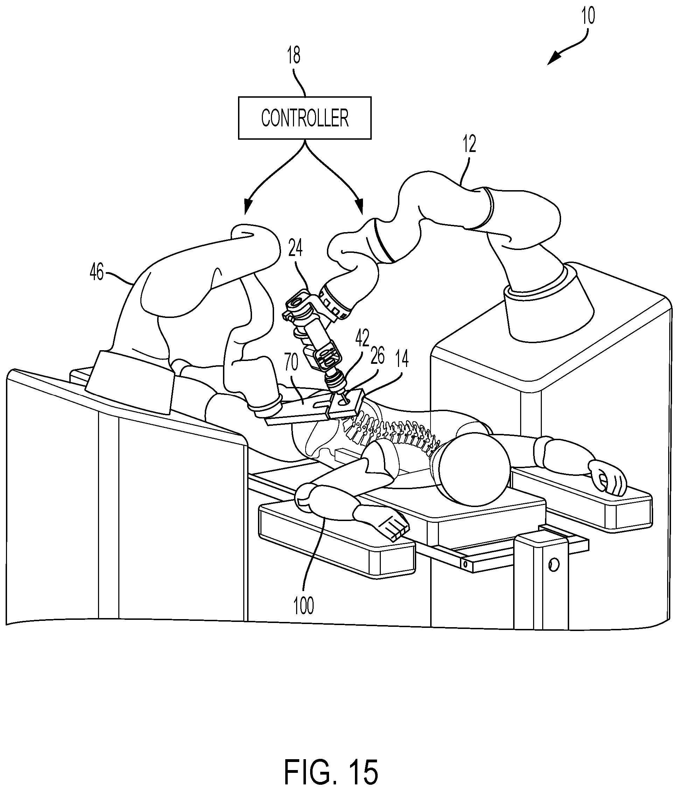

In accordance with another exemplary embodiment as shown in FIGS. 15-17, the subject disclosure provides a robotic surgical system having first and second robot arms 12, 46 operatively coupled to one another. While not being limited to a particular theory, the first robot arm 12 is attached at its distal end to a field generator bracket 70 attached to the distal end of the first robot arm. The field generator bracket 70 extends to attachment with the electromagnetic field generator 14. The second arm 46 includes a tool 26 attached to its distal end and disposed through the central aperture 44 of the field generator 14 attached to the field generator bracket 70 at the distal end of the first robot arm 12.

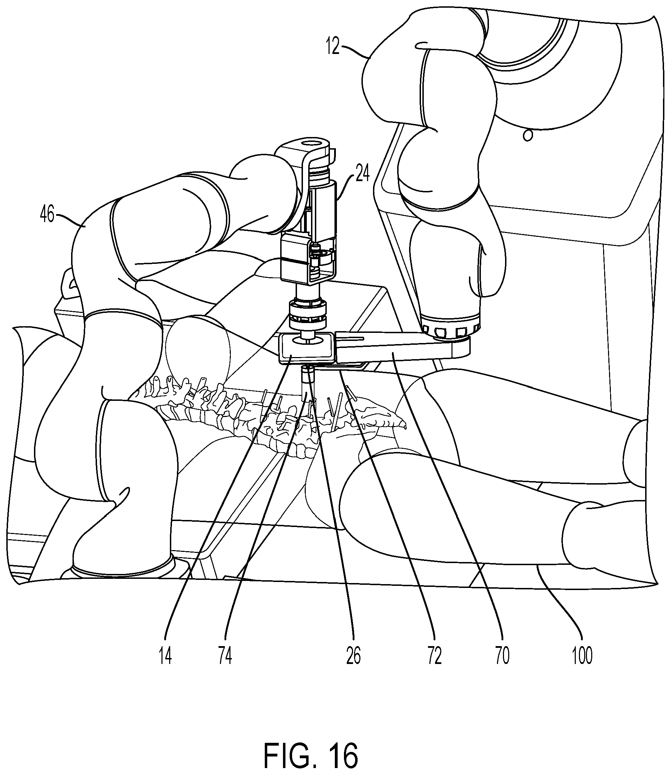

As can be seen in FIGS. 15-17, the first and second robot arms 12, 46 are operatively coupled to one another via a tool *e.g.,* a surgical retractor holder 72 and a surgical retractor 74. The surgical retractor holder 72 is attached to an underside of the field generator bracket 70, which extends from a distal end of the first robot arm 12. The surgical retractor holder extends beyond the field generator bracket and holds a surgical tool, for example, the surgical retractor 74 (FIG. 16) in alignment with the central aperture 44 of the field generator 14. The end effector 24 attached to the second arm 46 includes the tool 26 that is shown disposed through the central aperture 44 of the field generator into attachment with the surgical retractor 74 disposed at the distal end of the first robot arm 12 under the central aperture.

The data processing device or controller 18 of the robotic surgical system 10 is operatively in communication with the robotic end effector 24, field generator 14, field sensor 16 and tracking system 62, 92 having a camera 64. The controller is configured to receive and/or transmit information from the camera 64, field sensors 16, trackable objects and/or the robotic end effector 24. Additionally, the controller 18 is configured to determine a position of the sensors and/or trackable objects within the surgical working area based on data received from at least one of the field sensor, trackable object, and camera. For example, the controller is configured to determine a position of the sensor in relation to or relative to the robot arm 12 using data from one or more of the field sensor and the camera in a robot system such as that depicted in FIGS. 1-17, and described in greater detail below. As another example, the controller is configured to track both a first trackable object on the anatomy of a patient *e.g.,* on a patient's bone being worked on, such as a vertebral body, and a second trackable object attached to a tool or end effector on one or both robot arms to track the position of the anatomy itself relative to the tool/end effector. This is beneficial to keep the tool optimally aligned to the anatomy being worked on. Integrating the field generator with the second robot arm 46 allows the field generator effective volume to be optimally positioned relative to the active trackable objects, including at least one on the anatomy and another one on the tool/end effector.

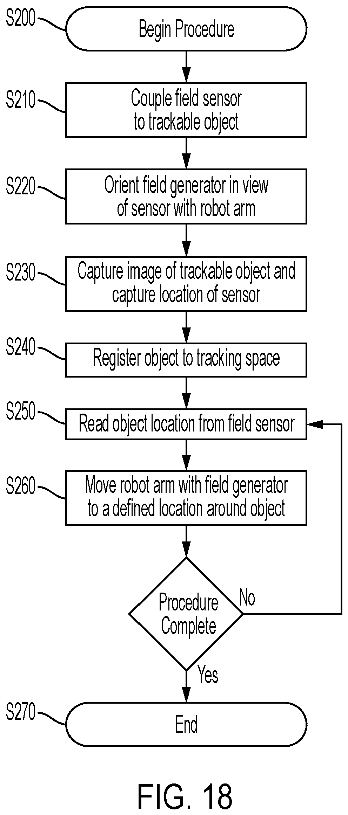

FIGS. 18-21 illustrate flowcharts of exemplary methods for conducting robotic spinal surgery with magnetic/electromagnetic tracking. Referring to FIG. 18, in accordance with another exemplary embodiment, in operation a surgical procedure of the subject disclosure utilizing the robotic surgical system with a field generator on the robot arm begins at Step S200 on a patient who is placed on a surgical table 108 in the clinical working area. At Step S210, a field sensor 16 is coupled to a trackable object. For example, the sensor may be coupled to a trackable object (*e.g.,* prosthesis, spinal pedicle screws, pins, clamps, screws, fasteners, tools, or other structure within the operating environment) via attachment (*e.g.,* adhesive, molded, soldered, press fit, embedded, sprayed, deposition) to the trackable object. Operation proceeds to Step S220, where a field generator 14 is oriented in view of the field sensor using a robot arm 12. At Step S230, a camera 64 captures an image of the trackable object and the location of the field sensor 16 in the clinical working area.

The trackable object is then registered to a tracking space adjacent the patient at Step S240. Operation proceeds to Step S250, where the navigation system, including a controller 18, the field generator 14 and/or the camera 64, determines the location of the trackable object from the sensor 16. The robot arm 12 is then moved with the field generator to a desired location around the trackable object at Step S260. The desired field generator location may be determined based on a variety of considerations. For example, the desired location of the field generator is at a location where the field generator 14 does not impede movement and operation of the robotic surgical system end effector 24 and any tools 26 performing the surgical procedure. In addition, the field generator should be positioned to maximize the effectiveness of any field generated effected volume 50 by the field generator where the surgical tools and end effector are performing operations in the surgical field and adjacent trackable objects, field sensor and fiducials.

If the procedure is completed, the surgical procedure ends at Step S270. For example, a surgical procedure may end when a number of pedicle screws have been implanted. Here, the process may include implanting a pedicle screw at a particular pedicle. This process may include steps to "burr" the cortical bone, drill a pedicle, tap the pedicle, and insert the pedicle screw in the pedicle. Each step may include an approach to the bone, cutting action and retraction of a tool to a "safe" position. At the point that the tool is retracted to the safe position, the robotic surgical system would pause and allow surgeon input as to whether the step has been done correctly and either move on to the next step or stop and make corrections. At the end of the process of implanting the pedicle screw, the robotic surgical system would pause and wait for further input from the surgeon to, for example, move on to a next pedicle whereupon the process of implanting a pedicle screw repeats. From the perspective of the robotic surgical system, the surgical procedure may end upon completion of the pedicle screw implantation.

However, if the procedure is not completed, the location of the trackable object is again read and determined from the sensor at Step S250, and the robot arm is moved to another desired location as needed based on the location of the trackable object at Step S260 until the procedure is completed at Step S270.

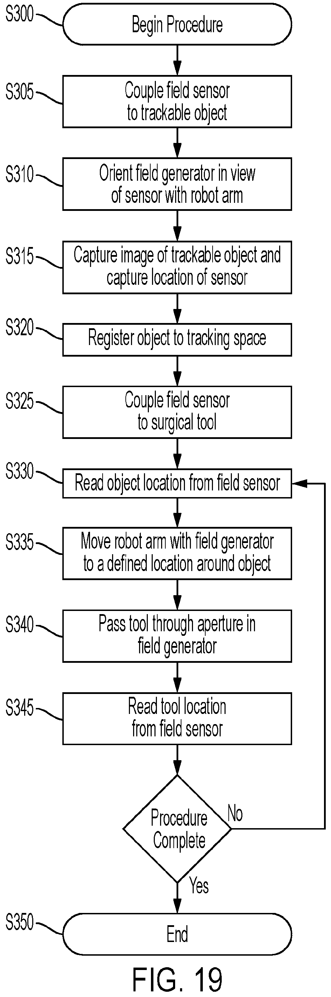

Referring to FIG. 19, in accordance with another exemplary embodiment, in operation a surgical procedure of the subject disclosure utilizing the robotic surgical system with a field generator on the robot arm, wherein the field generator has an aperture that a surgical tool can pass through begins at Step S300 on a patient who is placed on the surgical table 108. At Step S305, a field sensor 16 is coupled to a trackable object. Next, the field generator 14 is oriented in view of the sensor using the robot arm 12 at Step S310. At Step S315, a camera 64 captures an image of the trackable object and the location of the attached sensor 16 in the clinical working area. The trackable object is then registered to a tracking space adjacent the patient at Step S320.

Next, the sensor 16 is coupled to the surgical tool at Step S325. Operation proceeds to Step S330, where the navigation system, including a controller 18, the field generator 14 and/or the camera 64, reads and determines the location of the trackable object from the sensor 16. Based on the determination, the robot arm 12 may then be moved with the field generator 14 to a desired location around the trackable object at Step S335. Next, the tool is passed through the aperture 44 in the field generator at Step S340. Operation proceeds to Step S345, where the navigation system, including the controller 18 the field generator 14 and/or the camera 64, reads and determines the location of the tool from the sensor 16. If the procedure is completed, the surgical procedure ends at Step S350. However, if the procedure is not completed, then operation returns to Step S330, where the navigation system again reads and determines the location of the trackable object from the sensor 16, and the remaining Steps S335-S345 are repeated until the procedure is completed at Step S350.

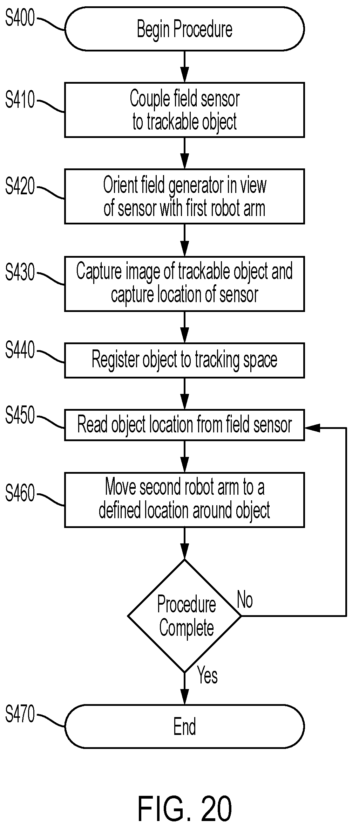

Referring to FIG. 20, in accordance with another exemplary embodiment, a surgical procedure of the subject disclosure utilizing the robotic surgical system with two robot arms and a field generator on one of the robot arms begins at Step S400 on a patient who is placed on the surgical table 108. At Step S410, a field sensor 16 is coupled to a trackable object. At Step S420, a field generator 14 is oriented in view of the sensor using the robot arm 12. At Step S430, a camera 64 captures an image of the trackable object and the location of the attached sensor 16 in the clinical working area.

The trackable object is then registered to a tracking space adjacent the patient at Step S440. Operation proceeds to Step S450, where the navigation system, including a controller 18, the field generator 14 and/or the camera 64, reads and determines the location of the trackable object from the sensor 16. Based on the determination, a second robot arm 46 is moved to a desired location around the trackable object at Step S460. Similar to Step S260 discussed above, the desired location of the field generator 14 is determined as a position where it does not impede movement and operation of the robotic surgical system end effector 24 and tools 26 performing the surgical procedure, and where it maximizes the effectiveness of its field generated effected volume. For example, if the first arm 12 and end effector 24 is drilling into one of the L3 pedicles, there would be a fiducial (*e.g.,* RF tracker) attached to the L3 vertebra and another fiducial attached to the end effector on the first robot arm. The field generator 14 on the end of robot arm 46 would ideally be positioned so that the magnetic field is optimized to detect the positions of both trackers and ultimately the position of the tool being used to perform the operation relative to the drilled pedicle. If the procedure is completed, the surgical procedure ends at Step S470, however, if the procedure is not completed, then operation returns to Step S450, where the location of the trackable object is again read and determined from the sensor, and the remaining steps are repeated until the procedure is completed at Step S470.

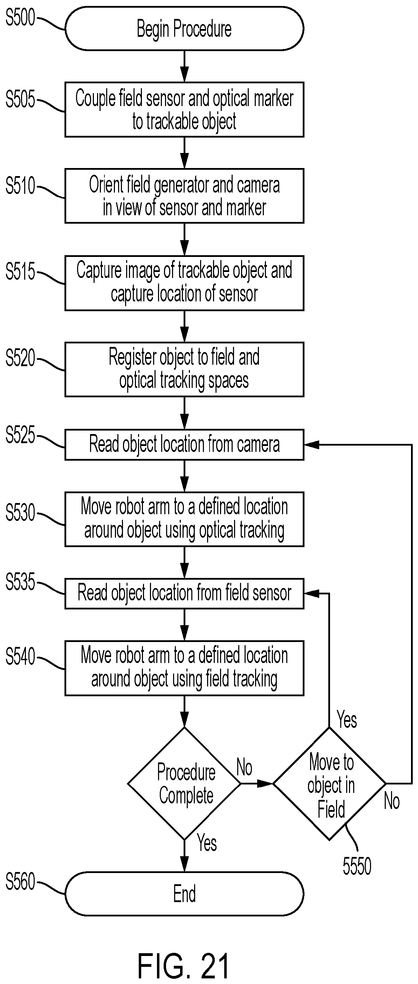

Referring now to FIG. 21, in accordance with yet another exemplary embodiment, a surgical procedure for a robot system w/ field generator and camera used for magnetic and optical tracking of field sensors that may be coupled to multiple trackable objects begins at Step S500 on a patient who is placed on the surgical table 108. At Step S505, a field sensor 16 and an optical fiducial are coupled to a trackable object. Operation proceeds to Step S510, where a field generator 14 and a camera 64 are orientated in view of the sensor and the optical fiducial. At Step S515, the navigation system, including the controller 18, the field generator 14 and the camera 64, captures an image of the trackable object and the location of the attached sensor 16 in the clinical working area.

Operation proceeds to Step S520, where the trackable object is registered to the field generated by the field generator 14 and the optical tracking spaces in the clinical working area. Operation proceeds to Step S525, where the location of the trackable object is read via the camera. Next, at Step S530, the robot arm is moved to a desired location around the trackable object using optical tracking via the camera and controller. The location of the trackable object within the field generator's effective volume is then read and determined via the sensor and controller at Step S535.

Operation continues to Step S540, where the robot arm 12 is moved to a desired location around the object using field tracking via the navigation system field generator, sensor and controller. If the procedure is complete, the surgical procedure ends at Step S560, however, if the procedure is not completed, the controller asks the user whether the robot arm should be moved to the trackable object in the field at Step S550. If the answer is yes, operation returns to Step S535 where the controller determines the trackable object location within the field generator effective volume from the sensor, and the robot arm is moved as needed to a desired location based on the location of the trackable object at Step S540 before the system inquires if the procedure is completed. If the answer is no, that is, if the robot arm should not be moved to the trackable object in the field, then operation returns to Step S525, where the location of another trackable object is read via the camera and controller. Then, again, the remaining steps are followed until the procedure is completed at Step S560.

The exemplary depicted sequence of executable method steps represents one example of a corresponding sequence of acts for implementing the functions described in the steps. The exemplary depicted steps may be executed in any reasonable order to carry into effect the objectives of the disclosed embodiments. No particular order to the disclosed steps of the method is necessarily implied by the depiction in FIGS. 18-21, and the accompanying description, except where any particular method step is reasonably considered to be a necessary precondition to execution of any other method step. Individual method steps may be carried out in sequence or in parallel in simultaneous or near simultaneous timing. Additionally, not all of the depicted and described steps need to be included in any particular scheme according to disclosure.

One of numerous benefits of the exemplary embodiments of the robotic surgical system described herein includes an improvement in the accuracy and precision of the placement of prosthesis, e.g., spinal pedicle screws. It should be noted that the EM tracking system does not require line of site visualization and corresponding trackers are quite small and can be easily incorporated into tools, including close to the tip of the tool which can lead to improved accuracy. Additionally, EM systems are not susceptible to interference from surgical fluids or reflective instruments and can be configured as a wireless tracking. In addition, optical systems allow larger, measuring volume and are not susceptible to ferrous metals near the field as they are a form of wireless tracking using the optical markers. The inventors found that using a combination of optical and EM tracking allows registration of larger areas of the spine while switching to EM tracking can provide better accuracy on a small area of interest such as a single vertebral body at a time giving an overall improved system and outcome.

Features of the subject disclosure include but are not limited to:
1. A robotic surgical system comprising a robot arm; a field generator for generating a magnetic or electromagnetic field; a field sensor coupled to a trackable object trackable within the magnetic or electromagnetic field; and a controller configured to determine a position of the trackable object in relation to the robot arm based on data from the field sensor.
2. The robotic surgical system of feature 1, further comprising a camera configured for optical tracking of the trackable object and operatively in communication with the controller.
3. The robotic surgical system of feature 2, wherein the controller is configured to determine the position of the trackable object in relation to the robot arm based on data received from at least one of the field sensor and the camera.
4. The robotic surgical system of feature 3, wherein the field sensor includes an optical fiducial.
5. The robotic surgical system of feature 1, wherein the field generator includes a central aperture.
6. The robotic surgical system of feature 1, wherein the robot arm includes an end effector.
7. The robotic surgical system of feature 1, further comprising a second sensor coupled to a second trackable object, wherein one of the field sensor and the second sensor is operatively attached to a tool, and the other one of the field sensor and the second sensor is configured for attachment to a patient's bone, and
   wherein the controller is configured to track a position of the tool relative to the patient's bone.
8. A robotic surgical system comprising a robot arm having an end effector and
   a field generator for generating a magnetic or electromagnetic field, and wherein the field generator includes a central aperture; a field sensor coupled to a trackable object; and a processer configured to determine a position of the trackable object relative to the robot arm based on data received from the field sensor.
9. The robotic surgical system of feature 8, further comprising a camera configured for optical tracking of the trackable object, and wherein the controller is configured to determine a position of the trackable object in relation to the robot arm based on data received from at least one of the sensor and the camera.
10. The robotic surgical system of feature 8, wherein the end effector includes a mounting nozzle coupled with a sterile drape retained by a drape cap.
11. The robotic surgical system of feature 8, wherein the end effector includes a housing, a gear train within the housing, and a tool holder operatively connected to the gear train for rotational movement relative to the housing.
12. The robotic surgical system of feature 8, wherein the end effector includes a tool holder holding a tool coupled at a distal end of the end effector.
13. A robotic surgical system comprising a first robot arm having a field generator for generating a magnetic or electromagnetic field; a second robot arm; a sensor coupled to a trackable object and trackable within the magnetic or electromagnetic field; and a controller operatively in communication with the first robot arm, the second robot arm and the sensor and configured to determine a position of the trackable object in relation to the first and second robot arms based on data received from the sensor.
14. The robotic surgical system of feature 13, further comprising a camera configured for optical tracking the trackable object, and wherein the controller is configured to determine a position of the trackable object in relation to the first and second robot arms based on data received from at least one of the sensor and the camera.
15. The robotic surgical system of feature 13, wherein the sensor is a field sensor.
16. The robotic surgical system of feature 13, wherein the second robot arm includes an end effector, and the field generator includes a central aperture.
17. The robotic surgical system of feature 13, wherein the trackable object is a surgical retractor.
18. The robotic surgical system of feature 13, wherein the second robot arm includes a tool.
19. The robotic surgical system of feature 18, further comprising a second sensor attached to the tool, wherein the sensor coupled to the trackable object is configured for attachment to a patient's bone, and the controller is configured to track the position of the tool relative to the patient's bone.

It will be appreciated by those skilled in the art that changes could be made to the various aspects described above without departing from the broad inventive concept thereof. It is to be understood, therefore, that the subject application is not limited to the particular aspects disclosed, but it is intended to cover modifications within the spirit and scope of the subject application as disclosed above and below.

## Claims

1. A robotic surgical system (10) comprising a robot arm (12) and a controller (18), **characterized by**:
a field generator (14) for generating a magnetic or electromagnetic field;
a field sensor (16) coupled to a trackable object trackable within the magnetic or electromagnetic field; and
wherein the controller (18) is configured to determine a position of the trackable object in relation to the robot arm (12) based on data from the field sensor (16).

2. The robotic surgical system (10) of claim 1, wherein the field sensor (16) includes an optical fiducial.

3. The robotic surgical system (10) of any preceding claim, wherein the field generator (14) includes a central aperture (44).

4. The robotic surgical system (10) of any preceding claim, wherein the robot arm (12) includes an end effector (24).

5. The robotic surgical system (10) of any preceding claim, further comprising a second sensor (16) coupled to a second trackable object, wherein one of the field sensor (16) and the second sensor (16) is operatively attached to a tool (26), and the other one of the field sensor (16) and the second sensor (16) is configured for attachment to a patient's bone,
wherein the controller (18) is configured to track a position of the tool (26) relative to the patient's bone.

6. The robotic surgical system (10) of any preceding claim, further comprising an end effector (24).

7. The robotic surgical system (10) of any preceding claim, further comprising a camera (64, 65, 75) configured for optical tracking of the trackable object, and wherein the controller (18) is configured to determine a position of the trackable object in relation to the robot arm (12) based on data received from at least one of the sensor (16) and the camera (64, 65, 75).

8. The robotic surgical system (10) of claim 6 or 7, wherein the end effector (24) includes a mounting nozzle (36) coupled with a sterile drape (38) retained by a drape cap (40).

9. The robotic surgical system (10) of claim 6 or 7, wherein the end effector (24) includes:
a housing (25),
a gear train (30) within the housing (25), and
a tool holder (42) operatively connected to the gear train (30) for rotational movement relative to the housing (25).

10. The robotic surgical system (10) of claim 6, wherein the end effector (24) includes a tool holder (42) holding a tool (26) coupled at a distal end of the end effector (24).

11. The robotic surgical system (10) of any preceding claim, further comprising:
a second robot arm (46), and
wherein the controller (18) is operatively in communication with the robot arm (12), the second robot arm (46) and the field sensor (16) and configured to determine a position of the trackable object in relation to the robot arm (12) and the second robot arm (46) based on data received from the field sensor (16).

12. The robotic surgical system (10) of claim 11, further comprising a camera (64, 65, 75) configured for optical tracking the trackable object, and wherein the controller (18) is configured to determine a position of the trackable object in relation to the robot arm (12) and the second robot arm (46) based on data received from at least one of the field sensor (16) and the camera (64, 65, 75).

13. The robotic surgical system (10) of claim 11 or 12, wherein the second robot arm (46) includes an end effector (24), and the field generator (14) includes a central aperture (44).

14. The robotic surgical system (10) of any one of claims 11-13, further comprising a tool (26) attached to the second robot arm (46), and a second field sensor (16) attached to the tool (26), wherein the field sensor (16) is coupled to the trackable object for attachment to a patient's bone, and the controller (18) is configured to track the position of the tool (26) relative to the patient's bone.

15. The robotic surgical system (10) of any preceding claim, wherein the trackable object is at least one of a surgical retractor (74), a spinous process tracking pin (52), an iliac tracking pin (54), an end effector (24), a tool (26), a spinous process (55), an ilium (56), and a vertebral body (58).
